# EUROPEAN PATENT SPECIFICATION

(11) **EP 4 144 406 B1**
(45) Date of publication and mention of the grant of the patent: **01.07.2026**
(21) Application number: 22194070.3
(22) Date of filing: 06.09.2022
(51) Int. Cl.: A61N 1/36, A61N 1/05, A61B 5/00

(54) **CLOSED LOOP CONTROL OF TIBIAL NERVE STIMULATION AND EFFICACY MONITORING**
STEUERUNG MIT GESCHLOSSENEM REGELKREIS DER NERVENSTIMULATION EINES SCHIENBEINS UND WIRKSAMKEITSÜBERWACHUNG
COMMANDE EN BOUCLE FERMÉE DE STIMULATION NERVEUSE TIBIALE ET DE SURVEILLANCE DE L'EFFICACITÉ

(30) Priority: 07.09.2021 US 202163241514 P; 29.07.2022 US 202217816153
(43) Date of publication of application: 08.03.2023
(73) Proprietor: Medtronic, Inc., Minneapolis, MN 55342-5604 (US)
(72) Inventor: Offutt, Sarah, Minneapolis, 55432-3568 (US); Slopsema, Julia, Minneapolis, 55432-3568 (US); Johnson, Katelynn, Minneapolis, 55432-3568 (US)
(74) Representative: Zimmermann & Partner Patentanwälte mbB

(56) References cited:
- WO-A1-2011/112843
- US-A1- 2003 153 957
- US-A1- 2016 303 376
- US-A1- 2018 078 769
- US-A1- 2018 126 169

## Description

### TECHNICAL FIELD

The disclosure relates to implantable medical devices configured to deliver electrical stimulation therapy.

### BACKGROUND

Medical devices may be external or implanted and may be used to deliver electrical stimulation therapy to patients via various tissue sites to treat a variety of symptoms or conditions such as chronic pain, tremor, Parkinson's disease, epilepsy, urinary or fecal incontinence, urinary frequency, urinary retention, sexual dysfunction, obesity, or gastroparesis. A medical device may deliver electrical stimulation therapy via one or more leads that include electrodes located proximate to target locations associated with the brain, the spinal cord, pelvic nerves, peripheral nerves, or the gastrointestinal tract of a patient. Stimulation proximate the spinal cord, proximate the sacral nerve, within the brain, and proximate peripheral nerves are often referred to as spinal cord stimulation (SCS), sacral neuromodulation (SNM), deep brain stimulation (DBS), and peripheral nerve stimulation (PNS), including tibial nerve stimulation. Electrical stimulation may be delivered to a patient by the medical device in a train of electrical pulses, and parameters of the electrical pulses may include a frequency, an amplitude, a pulse width, on-time, off-time, and a pulse shape. An evoked compound action potential (ECAP) is synchronous firing of a population of neurons which occurs in response to the application of a stimulus including, in some cases, an electrical stimulus by a medical device. A local field potential (LFP) is a transient electrical signal generated in the nervous system and/or other tissues. Document US 2016/30337 A1 relates to a stimulation therapy device.

### SUMMARY

The invention is defined in the appended claims. In general, the disclosure describes techniques for closed loop control of electrical stimulation therapy by measuring a neuromuscular response (e.g., sensory and motor response) to determine that the electrical stimulation therapy is adequately stimulating the target nerve. In some examples, a system using the techniques of this disclosure may measure a reflex response, e.g., using electromyography (EMG), which measures muscle response or electrical activity in response to a nerve's stimulation of the muscle. In some examples, the techniques of this disclosure may measure an H-reflex, such as an evoked motor response that is caused by a first afferent stimulation and, through a synapse, an efferent alpha-motor neuron excitation.

In one example, this disclosure describes a system comprising at least one sensor configured to measure a motor response of a patient; and a device that includes electrical stimulation circuitry configured to output electrical stimulation therapy to a target nerve of the patient; and processing circuitry operatively coupled to the electrical stimulation circuitry, the processing circuitry configured to: receive an indication from the at least one sensor indicating the motor response; measure a time interval between an output of an electrical stimulation therapy event from the electrical stimulation circuitry and the received indication of the motor response; determine whether the received indication of the motor response is valid based on the measured time interval; responsive to determining that the motor response is valid, determine whether a magnitude of the received indication of the motor response satisfies a limit; responsive to the magnitude of the received indication of the motor response failing to satisfy the limit, cause the electrical stimulation circuitry to adjust at least one parameter of a subsequent electrical stimulation therapy event.

In another example, this disclosure describes a device comprising electrical stimulation circuitry configured to output electrical stimulation therapy to a target nerve of the patient; and processing circuitry operatively coupled to the electrical stimulation circuitry, the processing circuitry configured to: receive an indication from at least one sensor indicating the motor response; measure a time interval between an output of an electrical stimulation therapy event from the electrical stimulation circuitry and the received indication of the motor response; determine whether the received indication of the motor response is valid based on the measured time interval; responsive to determining that the motor response is valid, determine whether a magnitude of the received indication of the motor response satisfies a limit; responsive to the magnitude of the received indication of the motor response failing to satisfy the limit, cause the electrical stimulation circuitry to adjust at least one parameter of a subsequent electrical stimulation therapy event.

**In** another example, this disclosure describes a method comprising delivering, by electrical stimulation circuitry of an implantable medical device, electrical stimulation therapy to a target nerve of a patient; receiving, by processing circuitry of the implantable medical device, an indication from at least one sensor indicating a motor response of the patient; measuring, by the processing circuitry, a time interval between the delivered electrical stimulation therapy from the electrical stimulation circuitry and the received indication of the motor response; determining, by the processing circuitry, whether the received indication of the motor response is valid based on the measured time interval; responsive to determining that the motor response is valid, determining, by the processing circuitry, whether a magnitude of the received indication of the motor response satisfies a limit; responsive to the magnitude of the received indication of the motor response failing to satisfy the limit, causing, by the processing circuitry, the electrical stimulation circuitry to adjust at least one parameter of a subsequent electrical stimulation therapy event.

The details of one or more examples of the disclosure are set forth in the accompanying drawings and the description below. Other features, objects, and advantages of the disclosure will be apparent from the description and drawings, and from the claims.

### BRIEF DESCRIPTION OF DRAWINGS

FIG. 1 is a conceptual diagram illustrating an example system including a medical device and external computing devices according to one or more techniques of this disclosure.
FIG. 2 is a block diagram illustrating example components of the medical device of FIG. 1.
FIG. 3 is a block diagram of an example external computing device of FIG. 1.
FIG. 4 is a conceptual diagram illustrating features of a medical device according to one or more techniques of this disclosure.
FIG. 5A is a timing diagram illustrating an example stimulation delivery and measured response according to one or more techniques of this disclosure.
FIG. 5B is a graph illustrating how the measured response may change based on the stimulation intensity.
FIG. 6 is a flow chart illustrating an example operation of the system of this disclosure.

### DETAILED DESCRIPTION

The disclosure describes techniques for closed loop control of electrical stimulation therapy by measuring a neuromuscular response (e.g., sensory and/or motor response) to determine that the electrical stimulation therapy is adequately stimulating the target nerve. In some examples, a system according to the techniques of this disclosure may measure a reflex response, e.g., using electromyography (EMG), which measures muscle response or electrical activity in response to a nerve's stimulation of the muscle. A system of this disclosure may include an implantable or wearable medical device. In some examples, a system using the techniques of this disclosure may measure an H-reflex, such as an evoked motor response that is caused by a first afferent stimulation, through a signal synapse, and an efferent alpha-motor neuron excitation. In some examples, the system may measure an M-wave, in which the action potentials may travel directly toward the muscle.

In some examples, a medical device configured for tibial nerve stimulation may use the techniques of this disclosure to determine that the tibial nerve stimulation is adequately stimulating the tibial nerve. For example, a measured EMG, within a specific time window related to nerve conduction up and down the leg at specific locations (e.g., great toe, lesser toes), may indicate that the medical device adequately stimulated the nerve. A nerve may include both sensory fibers and motor fibers and stimulating the nerve may include includes stimulating either or both sensory and/or motor fibers. The EMG signal may be measured by the medical device that delivered the stimulation or by a secondary sensor placed on the foot or as part of a recharge cuff. Additionally, the strength of the response over time may indicate the plasticity that repeated stimulation may induce and thereby provide an indication relating to overall effectiveness of therapy. In some examples, a medical device configured for closed loop therapy delivery may alter the parameters of the therapy event, e.g., stimulation level, pulse width, pulse pattern or other parameters dependent on the amplitude or other characteristics of the H-reflex, M-wave or other measured bioelectrical signals. In particular, the measured EMG response related to the H-reflex may be directly related to the nerve's sensory fibers captured. The size of the H-reflex in response to the stimulation could allow stimulation to be increased and decreased, and additionally over time, the H-reflex may be a way to measure plasticity associated with effective therapy.

In some examples, the medical system of this disclosure may operate as a fully automatic closed loop system. In other examples, the system of this disclosure may operate as partially manually controlled and partially automatic controls.

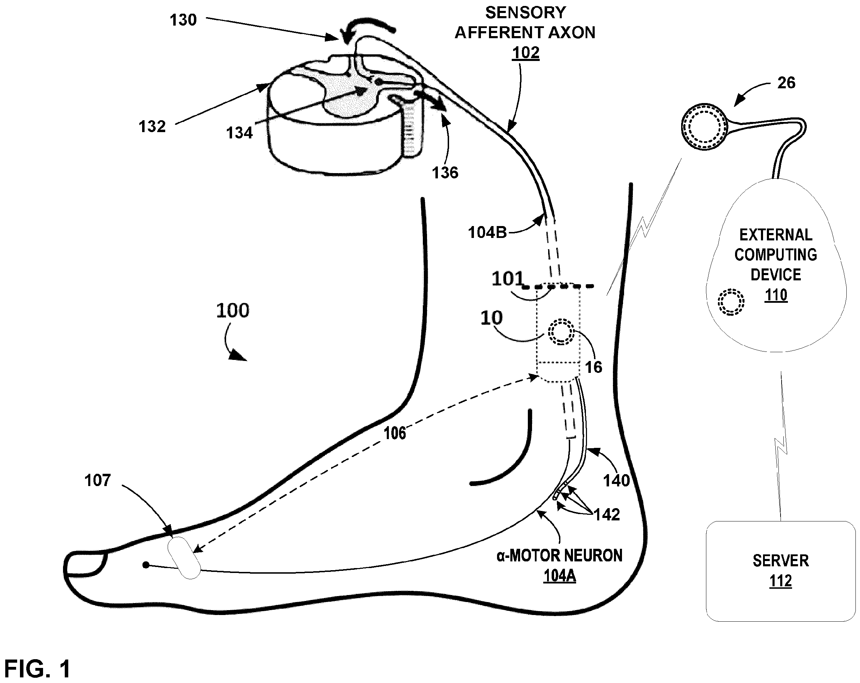

FIG. 1 is a conceptual diagram illustrating an example system including a medical device and external computing devices according to one or more techniques of this disclosure. The example of system 100 provides closed loop control of tibial nerve stimulation used to treat certain patient disorders. Other open loop nerve stimulation systems may initially rely on a patient's perceived motor response or sensation during initial setup and programming after implanting, or attaching, a nerve stimulation device to the patient. A clinician may question the patient while adjusting settings on the nerve stimulation device to determine that the tibial nerve stimulation is adequately stimulating the tibial nerve. The clinician may then set one stimulation level for that patient. However, as a patient moves around or changes foot position, the target nerve can move, e.g., deeper into the leg, and therefore the set stimulation level may no longer be adequate, or be too high and cause discomfort. Thus, the closed loop techniques of system 100 may help determine adequate nerve capture, e.g., a therapy event that exceeds the nerve capture threshold and stimulates either or both of sensory fibers and/or motor fibers of the nerve.

The example of system 100 in FIG. 1 includes an implantable medical device 10, external computing device 110, one or more servers 112, and one or more external sensors 107. Implantable medical device 10 (IMD 10), may be placed via incision 101 such that one or more electrodes connected to IMD 10 may stimulate and/or sense a tibial nerve, such as sensory afferent axon 102 and alpha motor neurons 104A and 104B, also referred to as α-motor neurons 104A and 104B. In some examples, IMD 10 may connect to lead 140, which may include one or more electrodes 142. In other examples, such as leadless examples, the electrodes for IMD 10 may be attached to a housing of IMD 10. In other examples, system 100 may include some combination of both leadless electrodes and leads that include electrodes, e.g., lead 140. The example of FIG. 1 shows lead 140 with electrodes 142 placed near α-motor neurons 104A. In other examples lead 140 may be located to deliver therapy and/or sense bioelectrical signals at any other location, for example near the patient's toe, along α-motor neurons 104B, and so on.

In some examples, IMD 10 may be implanted outside of the fascia near the tibial nerve. In other words, IMD 10 may be implanted in a pocket between the skin and the fascia. The fascia may be located between the target nerve and the stimulation and sensing electrodes of IMD 10. Implantation outside the fascia may improve patient comfort and recovery because the fascia is not cut and does not need time to heal. However, the fascia located between the electrodes and the target nerve, may result in reduced sensitivity for sensing and or therapy deliver. For example, therapy delivery circuitry of IMD 10 may be configured with higher stimulation intensity to be able to capture the nerve. In some examples, higher stimulation intensity may result in shorter battery life and/or more recharging sessions required. Based on the implant location, the delivered therapy event may be considered as stimulation near a portion of the tibial nerve.

IMD 10 may include processing circuitry, electrical stimulation circuitry, sensing circuitry, recharging circuitry, communication circuitry and so on (not shown in FIG. 1). Recharging circuitry and/or communication circuitry of IMD 10 may be connected to antenna 16. Though the example of FIG. 1 describes an implantable device, the techniques of this disclosure may also apply to a wearable nerve stimulation system, which may include a power supply external to the patient. Also, recharging circuitry may not be needed in examples where a non-rechargeable primary power cell is used for power.

Sensor 107 may be one of several sensors in communication with IMD 10 via communication channel 106. Sensor 107 may be implanted or worn externally, e.g., in a sock, sleeve, belt, adhesive and so on. Communication channel 106 may be wired or wireless and sensors, including 107 may also be wired or wireless. In some examples, one or more sensors (e.g., include sensor 107) may also communicate with external computing device 110. In some examples, the sensors may communicate indirectly with IMD 10 via external computing device 110. In some examples, sensor 107 sensor may be configured to measure an electromyography (EMG) response, e.g., a neuromuscular response. In some examples, EMG and ECAPs signal capture in the lower leg region presents sensing challenges. For example, for electrodes implanted with fascia or other tissue between the electrodes and the target nerve, the sensing circuitry may have difficulty reliably measuring ECAPs signals. Measuring EMG signals, ECAP signals or other bioelectrical signals e.g., with a separate sensor like sensor 107, may help overcome these challenges.

External computing device 110 includes one or more antenna, such as antenna 26. External computing device 110 may be used to program or adjust settings of IMD 10 and may also recharge an electrical energy storage device, such as a battery, of IMD 10. External computing device 110 may also communicate with servers 112.

IMD 10 may be configured to treat impaired physiological functions of a patient, such as where disease, age, and injury may impair the physiological functions of a patient. In one example, bladder dysfunction, such as overactive bladder, urgency, or urinary incontinence, is a problem that may afflict people of all ages, genders, and races. Various muscles, nerves, organs and conduits within the pelvic floor cooperate to collect, store, and release urine. A variety of disorders may compromise urinary tract performance, and contribute to an overactive bladder, urgency, or urinary incontinence that interferes with normal physiological function. System 100 may help relieve some symptoms of some disorders.

Urinary incontinence may include urge incontinence and stress incontinence. In some examples, urge incontinence may be caused by disorders of peripheral or central nervous systems that control bladder micturition reflexes. Some patients may also suffer from nerve disorders that prevent proper triggering and operation of the bladder, sphincter muscles or nerve disorders that lead to overactive bladder activities or urge incontinence. In some cases, urinary incontinence may be attributed to improper sphincter function, either in the internal urinary sphincter or external urinary sphincter.

One type of therapy for treating bladder dysfunction includes delivery of electrical stimulation to a target tissue site within a patient to cause a therapeutic effect during delivery of the electrical stimulation. For example, delivery of electrical stimulation from IMD 10 to a target therapy site, e.g., a tissue site that delivers stimulation to modulate activity of a tibial nerve, spinal nerve (e.g., a sacral nerve), a pudendal nerve, dorsal genital nerve, an inferior rectal nerve, a perineal nerve, or branches of any of the aforementioned nerves, may provide a therapeutic effect for bladder dysfunction, such as a desired reduction in frequency of bladder contractions. In some cases, electrical stimulation of the tibial nerve may modulate afferent nerve activities to restore urinary function.

As one example, bladder dysfunction generally refers to a condition of improper functioning of the bladder or urinary tract, and may include, for example, an overactive bladder, urgency, or urinary incontinence. Overactive bladder (OAB) is a patient condition that may include symptoms, such as urgency, with or without urinary incontinence. Urgency is a sudden, compelling urge to urinate, and may often, though not always, be associated with urinary incontinence. Urinary incontinence refers to a condition of involuntary loss of urine, and may include urge incontinence, stress incontinence, or both stress and urge incontinence, which may be referred to as mixed urinary incontinence. As used in this disclosure, the term "urinary incontinence" includes disorders in which urination occurs when not desired, such as stress or urge incontinence. Other bladder dysfunctions may include disorders such as non-obstructive urinary retention.

In some examples, the techniques described in this disclosure are directed to delivery of neurostimulation therapy in a non-continuous manner which may include on-cycles and off-cycles. For example, an IMD may deliver neurostimulation therapy for a specified period of time followed by a specified period of time when the IMD does not deliver neurostimulation (e.g., withholds delivery of neurostimulation). A period during which stimulation is delivered (an on-cycle) may include on and off periods (e.g., a duty cycle or bursts of pulses) with short inter-pulse durations of time when pulses are not delivered. In some examples, on-cycles of therapy may be effective during certain times of the day, or under certain conditions, e.g., when the motor response is within a certain range, when the patient is moving, or alternatively, when the patient is not moving, or other conditions. IMD 10 may periodically check the motor response, such as at specified times of the day, and may determine that the measured conditions are within a target range to deliver therapy. In some examples, IMD 10 may automatically begin an on-cycle. In some examples, IMD 10 may signal the patient, e.g., via an external computing device such as a smart watch, smart phone or external computing device 110, that a therapy on-cycle is about to begin.

In some examples, system 100 may use the Hoffmann reflex (H-reflex) to provide feedback to IMD 10 that the delivered electrical stimulation therapy has captured the target nerve of the patient. The term "captured" in this disclosure means that an electrical stimulation therapy event generates a response in the target nerve. In some examples, parameters of the delivered electrical stimulation therapy, e.g., magnitude of voltage or current, pulse width, frequency and so on, may not generate a response in the target nerve because, for example, the magnitude of a pulse is too low, the electrode is too far from the target nerve, etc.

The H-reflex is an electrically induced reflex in contrast to the mechanically induced spinal stretch reflex. The H-reflex may bypass the muscle spindle and may act as an estimate of alpha motor neuron (aMN or αMN) excitability. The example of FIG. 1 depicts both H-reflex and muscle response (M-wave) pathways, e.g., a neuromuscular response. For the H-reflex, when IMD 10 delivers a sufficient short-duration, low-intensity electric stimulus to the tibial nerve, the electrical stimulus may elicit action potentials 130 in type Ia sensory afferents because sensory afferent axons such as sensory afferent axon 102 may have a large axon diameter.

For the H-reflex pathway, these action potentials may travel to the spinal cord 132, where the action potential may cause excitatory postsynaptic potentials 134, in turn eliciting action potentials 136, which travel down the alpha motor neuron (αMN) axons, e.g., 104B and 104A toward a muscle, such as a muscle controlling the toe of the patient. Alpha-motor neurons are the nerve fibers that directly innervate skeletal muscles to initiate contractions. In this manner a sensor, such as sensor 107 may measure a volley of efferent action potentials in the muscle as an H-reflex.

However, increasing the stimulus intensity of the electrical stimulation therapy event may causes action potentials to occur in the thinner axons of the αMNs, e.g., 104A. For the M-wave pathway, the action potentials may travel directly toward the muscle and be recorded as the M-wave. At the same time, action potentials from the higher intensity stimulus may propagate antidromically (backward) in the αMN 104B toward the spinal cord 132 to collide with action potentials of the evoked reflex response 136, thereby resulting in partial cancellation of the reflex response. For higher intensity stimulation orthodromic (toward the muscle) and antidromic (toward the spinal cord) action potentials may occur in MN axons. Orthodromic (toward the muscle) potentials may reach a maximum (Mmax), while antidromic (toward the spinal cord) action potentials may results in complete cancellation of the H-reflex.

Therefore, in some examples, the closed loop functions of system 100 may be desirable to deliver electrical stimulation therapy at a sufficient intensity to provide a therapeutic effect that eases the patient's symptoms, yet with a stimulation intensity (e.g., magnitude, pulse width, pulse repetition rate, and so on) to elicit a measurable H-reflex (e.g., the M-wave does not cancel the H-reflex). In addition, delivering electrical stimulation therapy at lower levels may reduce power consumption and therefore a battery of IMD 10 may last longer, or need less recharging, which may be beneficial for the patient.

In this disclosure, afferent stimulation, in the context of nerves means conducting toward the central nervous system. In some examples, afferent may be used synonymously with sensory since the sensory fibers convey sensations back to the spinal cord/brain. The term, efferent means away and is typically used for motor fibers. Thus afferent stimulation means stimulation targeted at sensory fibers. In some examples, because of the size mixture of sensory/motor fibers (or afferent/efferent) it can be difficult to get specific activation.

In some examples, the at least one sensor is attached to a housing of IMD 10 that is in contact with tissue of the patient and that includes the electrical stimulation circuitry. That is, rather than having sensor 107 at a distance from IMD 10, a sensor like sensor 107 may be attached to the housing of IMD 10. For either distal sensor 107 or a sensor on the housing of IMD 10, IMD 10 may include sensing circuitry that measures a stimulation artifact that results from the delivered electrical stimulation therapy. Similarly, IMD 10 may be configured to detect evoked compound action potentials (ECAPs). The system may measure both the stimulation artifact and ECAP shortly after the delivered electrical stimulation therapy, e.g., less than about five millisecond (ms). In contrast, the H-reflex takes some time for the nerve impulse to travel to the spinal cord and back to the sensor, e.g., sensor 107, or a sensor on the housing of IMD 10. The time may be several milliseconds, such as between approximately fifteen and thirty milliseconds. Processing circuitry of IMD 10 may measure a time interval between an output of an electrical stimulation therapy event from the electrical stimulation circuitry and the received indication of the motor response to ensure that sensed motor response is actually a motor response, and not some other signal.

In addition, measuring the time interval may help ensure that the measured motor response is the H-reflex elicited by the delivered electrical stimulation therapy. In other words, the processing circuitry may determine whether the received indication of the motor response is valid based on the measured time interval. A measured signal that is not valid may be an inappropriate signal to use for a closed loop response. In some examples, a valid measured signal may be an H-reflex as reflex muscle response recorded in a muscle, e.g., a toe muscle measured by sensor 107. The valid H-reflex may be caused by a delivered electrical stimulation therapy event that causes an afferent stimulation in the target nerve. In other examples, processing circuitry of the medical device may measure and analyze some combination of the H-reflex, an M-wave, or ECAP to provide closed loop control of stimulation.

Responsive to determining that the motor response is valid, the processing circuitry of IMD 10 may determine whether a magnitude of the received indication of the motor response satisfies a limit, e.g., a target value store at a memory operatively coupled to the processing circuitry. The measured indication of the H-reflex may satisfy a limit when the magnitude is at least above a magnitude limit, or in other examples, when the magnitude is within a target range. In this closed loop technique, the stimulation therapy event may elicit an H-reflex, and processing circuitry of IMD 10 may compare the measured H-reflex to one or more stored values, i.e., targets. Based on the comparison, the processing circuitry may adjust parameters of subsequent stimulation therapy events. In some examples, each therapy event may elicit an H-reflex. In other examples, the processing circuitry may periodically elicit an H-reflex, e.g., every third therapy event, every tenth therapy event, and the like.

IMD 10 may also operate in other modes of closed loop control. In a second example technique, processing circuitry of IMD 10 may measure an indication of a motor response, e.g., an H-reflex or an M-wave. Processing circuitry of IMD 10 may decrement the output stimulation until the sensing circuitry of IMD 10 no longer detects the motor response. In this manner, IMD 10 may output an electrical stimulation therapy event, e.g., a pulse, burst, pattern of pulses, and so on, that is high enough to likely capture the tibial nerve, but just below the level to generate a motor response for the patient. Such a stimulation scheme, e.g., just below the motor response for the patient at a particular time, may have advantages such as minimizing battery power consumption while still capturing the target nerve, e.g., the tibial nerve, to provide effective therapy.

In a third example technique, processing circuitry for system 100, may receive an indication from sensing circuitry connected to the one or more electrodes of system 100 that detect an afferent response to an electrical stimulation therapy event. The processing circuitry may also receive an indication of the motor response, e.g., the H-reflex generated by the therapy event. The processing circuitry, e.g., of IMD 10 may adjust one or more parameters of the therapy event, e.g., magnitude, number of pulses in a burst, and so on, to ensure the therapy event elicits a response between the target range of stimulation intensity that elicits only an afferent response and a stimulation intensity that elicits a motor response. In other words, the processing circuitry may receive an indication of an H-reflex above a predetermined limit stored at a memory location, e.g., a limit of zero. The processing circuitry may adjust parameters of the electrical stimulation therapy event to ensure that the therapy event does not elicit an H-reflex, but does elicit an afferent response. Said another way, the processing circuitry may adjust the parameters of a subsequent therapy event to be above the capture threshold for an afferent response (e.g., elicit an afferent response) but less than the capture threshold for an H-reflex that comes from the spinal cord.

Responsive to the magnitude of the received indication of the motor response failing to satisfy the target, the processing circuitry of IMD 10 may cause the electrical stimulation circuitry to adjust at least one parameter of a subsequent electrical stimulation therapy event. For example, the electrical stimulation circuitry may increase or decrease the stimulation level, e.g., an output magnitude of a stimulation pulse, adjust the pulse width, adjust a frequency or pulse repetition rate, number of pulses in a burst, or some other characteristic.

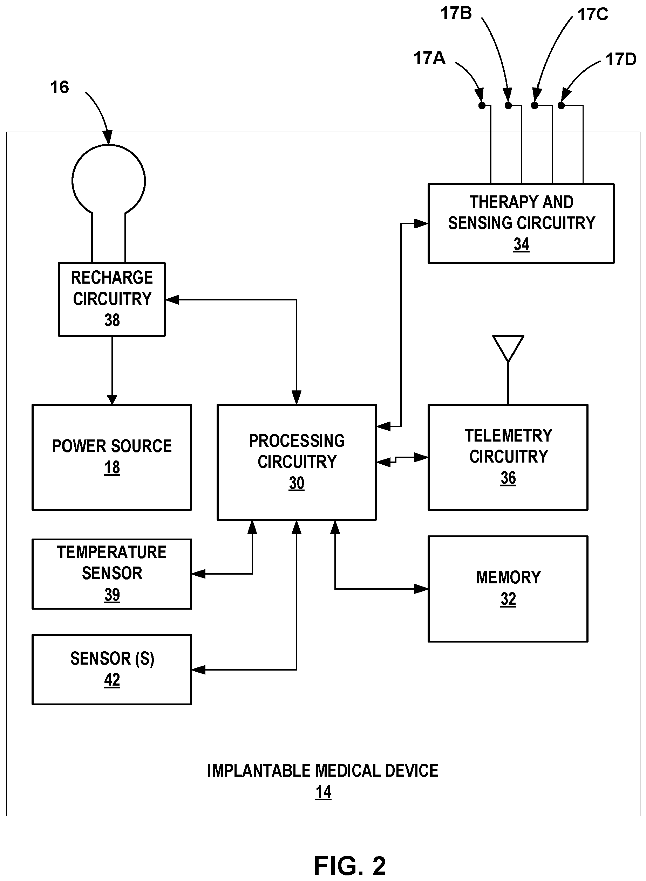

FIG. 2 is a block diagram illustrating example components of the medical device of FIG. 1. Implantable medical device 14 is an example of IMD 10 described above in relation to FIG. 1. In the example illustrated in FIG. 2, IMD 14 includes temperature sensor 39, coil 16, processing circuitry 30, therapy and sensing circuitry 34, recharge circuitry 38, memory 32, telemetry circuitry 36, and power source 18. In other examples, IMD 14 may include a greater or a fewer number of components, e.g., in some examples, IMD 14 may not include temperature sensor 39. In general, IMD 14 may comprise any suitable arrangement of hardware, alone or in combination with software and/or firmware, to perform the various techniques described herein attributed to IMD 14 and processing circuitry 30, and any equivalents thereof.

Processing circuitry 30 of IMD 14 may include one or more processors, such as one or more microprocessors, digital signal processors (DSPs), application specific integrated circuits (ASICs), field programmable gate arrays (FPGAs), or any other equivalent integrated or discrete logic circuitry, as well as any combinations of such components. IMD 14 may include a memory 32, such as random access memory (RAM), read only memory (ROM), programmable read only memory (PROM), erasable programmable read only memory (EPROM), electronically erasable programmable read only memory (EEPROM), flash memory, comprising executable instructions for causing the processing circuitry 30 to perform the actions attributed to this circuitry. Moreover, although processing circuitry 30, therapy and sensing circuitry 34, recharge circuitry 38, telemetry circuitry 36, and temperature sensor 39 are described as separate modules, in some examples, some combination of processing circuitry 30, therapy and sensing circuitry 34, recharge circuitry 38, telemetry circuitry 36, sensors 42 and temperature sensor 39 are functionally integrated. In some examples, processing circuitry 30, therapy and sensing circuitry 34, recharge circuitry 38, telemetry circuitry 36, sensors 42 and temperature sensor 39 correspond to individual hardware units, such as ASICs, DSPs, FPGAs, or other hardware units. In this disclosure, therapy and sensing circuitry 34 may be referred to as therapy circuitry 34, for simplicity.

Memory 32 may store therapy programs or other instructions that specify therapy parameter values for the therapy provided by therapy circuitry 34 and IMD 14. In some examples, memory 32 may also store temperature data from temperature sensor 39, instructions for recharging rechargeable power source 18, thresholds, limits, ranges and other values, instructions for communication between IMD 14 and an external computing device, or any other instructions required to perform tasks attributed to IMD 14. Memory 32 may be configured to store instructions for communication with and/or controlling one or more temperature sensors of temperature sensor 39. In various examples, memory 32 stores information related to determining the temperature of housing 19 and/or exterior surface(s) of housing 19 of IMD 14 based on temperatures sensed by one or more temperature sensors, such as temperature sensor 39, located within IMD 14.

**In** some examples, IMD 14 may also include one or more additional sensors 42. Sensors 42 may include one or more accelerometers, pressure sensors, and so one. **In** the example of accelerometers, sensors 42 may be sensitive to a gravitational field, and or sensitive to motion. Signals received from sensors 42 by processing circuitry 30 may provide information such as patient posture, activity level and so on. **In** some examples, processing circuitry 30 may process information from sensors 42 to inform therapy selection, starting or stopping therapy, to confirm or distinguish certain perceived data and so on, based on instructions stored at memory 32, or based on commands received by an external computing device.

For example, memory 32 may store programming settings such as electrical stimulation therapy output magnitude, pulse width, and so on. Memory 32 may store timing limits to determine whether a sensed bioelectrical signal is a valid H-reflex detected in response to an output electrical stimulation therapy event. Memory 32 may store programming instructions that when executed by processing circuitry 30 cause processing circuitry 30 to cause electrical stimulation circuitry therapy circuitry 34 to deliver electrical stimulation therapy to a target nerve of a patient. The programming instructions may also cause processing circuitry to receive the indication from at least one sensor indicating a motor response of the patient and measure a time interval between the delivered electrical stimulation therapy from the electrical stimulation circuitry and the received indication of the motor response. The processing circuitry may further determine whether the received indication of the motor response is valid based on comparing the measured time interval to the stored timing limits. Sensed bioelectrical signals outside of the expected timing window may be based on some other cause other than the electrical stimulation therapy.

Therapy and sensing circuitry 34 may generate and deliver electrical stimulation under the control of processing circuitry 30. In some examples, processing circuitry 30 controls therapy circuitry 34 by accessing memory 32 to selectively access and load at least one of the stimulation programs to therapy circuitry 34. For example, in operation, processing circuitry 30 may access memory 32 to load one of the stimulation programs to therapy circuitry 34. In such examples, relevant stimulation parameters may include a voltage amplitude, a current amplitude, a pulse rate, a pulse width, a duty cycle, or the combination of electrodes 17A, 17B, 17C, and 17D (collectively "electrodes 17") that therapy circuitry 34 may use to deliver the electrical stimulation signal as well as sense biological signals. In other examples, IMD 14 may have more or fewer electrodes than the four shown in the example of FIG. 2. In some examples electrodes 17 may be part of or attached to a housing of IMD 14, e.g., a leadless electrode. In other examples, one or more of electrodes 17 may be part of a lead implanted in or attached to a patient to sense biological signals and/or deliver electrical stimulation.

In some examples, one or more electrodes connected to therapy circuitry 34 may connect to one or more sensing electrodes, e.g., attached to housing of IMD 14. In some examples the electrodes may be configured to detect direct motor activity as well as the evoked motor response caused by the electrical stimulation therapy event. Processing circuitry 30 may determine whether the detected motor response is a valid efferent alpha motor neuron excitation through a spinal synapse, based on timing, as described above. In some examples, processing circuitry 30 may cause sensed bioelectrical signals, e.g., the raw measured signals, to be stored at memory 32. In some examples, processing circuitry 30 may analyze the stored signals to determine features of the signals, e.g., magnitude, latency, duration and so on. In some examples, processing circuitry 30 may cause all or a portion of the stored signals to be transmitted to external processing circuitry, e.g., server 112 depicted in FIG. 1, for further storage and analysis.

In some examples, processing circuitry 30 may control therapy circuitry 34 to deliver stimulation pulses continuously or periodically. For example, therapy circuitry 34 may deliver stimulation therapy for a ten minute session each day, thirty minutes each day, ten minutes twice a day, or some other delivery period, such as a sixty minutes every other day, or any other variation that may relieve the patient's symptoms. In some examples, processing circuitry may cause the sensing circuitry to measure a response, e.g., an H-reflex, at the beginning of each session, two or three times during a therapy session, after each stimulation pulse or other therapy event, every X minutes, or any other combination. In other examples, processing circuitry may receive input from the patient or other caregiver and operate as partially manually controlled and partially automatic control. In some examples, therapy circuitry 34 may delivery electrical stimulation therapy with a pulse width configurable from 2 to 500µs. Stimulation frequency be configurable from 2 to1200 Hz. Stimulation levels, e.g., stimulation magnitude, may range from 0.5 mA to 25mA. The selected stimulation intensity may depend in a particular patient, the implant location, the nerve location and nerve depth, e.g., depth of the nerve under the fascia, patient sensitivity, blood chemistry and other factors.

IMD 14 also includes components to receive power to recharge rechargeable power source 18 when rechargeable power source 18 has been at least partially depleted. As shown in FIG. 2, IMD 14 includes coil 16 and recharge circuitry 38 coupled to rechargeable power source 18. Recharge circuitry 38 may be configured to charge rechargeable power source 18 with the selected power level determined by either processing circuitry 30 or an external charging device, such as external computing device 110 described above in relation to FIG. 1. Recharge circuitry 38 may include any of a variety of charging and/or control circuitry configured to process or convert current induced in coil 16 into charging current to charge power source 18.

Secondary coil 16 may include a coil of wire or other device capable of inductive coupling with a primary coil disposed external to patient 12. Although secondary coil 16 is illustrated as a simple loop of in FIG. 2, secondary coil 16 may include multiple turns of conductive wire. Secondary coil 16 may include a winding of wire configured such that an electrical current can be induced within secondary coil 16 from a magnetic field. The induced electrical current may then be used to recharge rechargeable power source 18.

Recharge circuitry 38 may include one or more circuits that process, filter, convert and/or transform the electrical signal induced in the secondary coil to an electrical signal capable of recharging rechargeable power source 18. For example, in alternating current induction, recharge circuitry 38 may include a half-wave rectifier circuit and/or a full-wave rectifier circuit configured to convert alternating current from the induction to a direct current for rechargeable power source 18. The full-wave rectifier circuit may be more efficient at converting the induced energy for rechargeable power source 18. However, a half-wave rectifier circuit may be used to store energy in rechargeable power source 18 at a slower rate. In some examples, recharge circuitry 38 may include both a full-wave rectifier circuit and a half-wave rectifier circuit such that recharge circuitry 38 may switch between each circuit to control the charging rate of rechargeable power source 18 and temperature of IMD 14.

Rechargeable power source 18 may include one or more capacitors, batteries, and/or other energy storage devices. Rechargeable power source 18 may deliver operating power to the components of IMD 14. In some examples, rechargeable power source 18 may include a power generation circuit to produce the operating power. Rechargeable power source 18 may be configured to operate through many discharge and recharge cycles. Rechargeable power source 18 may also be configured to provide operational power to IMD 14 during the recharge process. In some examples, rechargeable power source 18 may be constructed with materials to reduce the amount of heat generated during charging. In other examples, IMD 14 may be constructed of materials and/or using structures that may help dissipate generated heat at rechargeable power source 18, recharge circuitry 38, and/or secondary coil 16 over a larger surface area of the housing of IMD 14.

Although rechargeable power source 18, recharge circuitry 38, and secondary coil 16 are shown as contained within the housing of IMD 14, in alternative implementations, at least one of these components may be disposed outside of the housing. For example, in some implementations, secondary coil 16 may be disposed outside of the housing of IMD 14 to facilitate better coupling between secondary coil 16 and the primary coil of external charging device. In other examples, power source 18 may be a primary power cell and IMD 14 may not include recharge circuitry 38 and recharge coil 16.

Processing circuitry 30 may also control the exchange of information with an external computing device using telemetry circuitry 36. Telemetry circuitry 36 may be configured for wireless communication using radio frequency protocols, such as BLUETOOTH, or similar RF protocols, as well as using inductive communication protocols. Telemetry circuitry 36 may include one or more antennas 37 configured to communicate with external charging device, for example. Processing circuitry 30 may transmit operational information and receive therapy programs or therapy parameter adjustments via telemetry circuitry 36. Also, in some examples, IMD 14 may communicate with other implanted devices, such as stimulators, control devices, or sensors, via telemetry circuitry 36. In addition, telemetry circuitry 36 may be configured to control the exchange of information related to sensed and/or determined temperature data, for example temperatures sensed by and/or determined from temperatures sensed using temperature sensor 39. In some examples, telemetry circuitry 36 may communicate using inductive communication, and in other examples, telemetry circuitry 36 may communicate using RF frequencies separate from the frequencies used for inductive charging.

In some examples, processing circuitry 30 may transmit additional information to external charging device related to the operation of rechargeable power source 18. For example, processing circuitry 30 may use telemetry circuitry 36 to transmit indications that rechargeable power source 18 is completely charged, rechargeable power source 18 is fully discharged, or any other charge status of rechargeable power source 18. In some examples, processing circuitry 30 may use telemetry circuitry 36 to transmit instructions to external charging device, including instructions regarding further control of the charging session, for example instructions to lower the power level or to terminate the charging session, based on the determined temperature of the housing/external surface 19 of the IMD.

Processing circuitry 30 may also transmit information to external charging device that indicates any problems or errors with rechargeable power source 18 that may prevent rechargeable power source 18 from providing operational power to the components of IMD 14. In various examples, processing circuitry 30 may receive, through telemetry circuitry 36, instructions for algorithms, including formulas and/or values for constants to be used in the formulas, that may be used to determine the temperature of the housing 19 and/or exterior surface(s) of housing 19 of IMD 14 based on temperatures sensed by temperature sensor 39 located within IMD 14 during and after a recharging session performed on rechargeable power source 18.

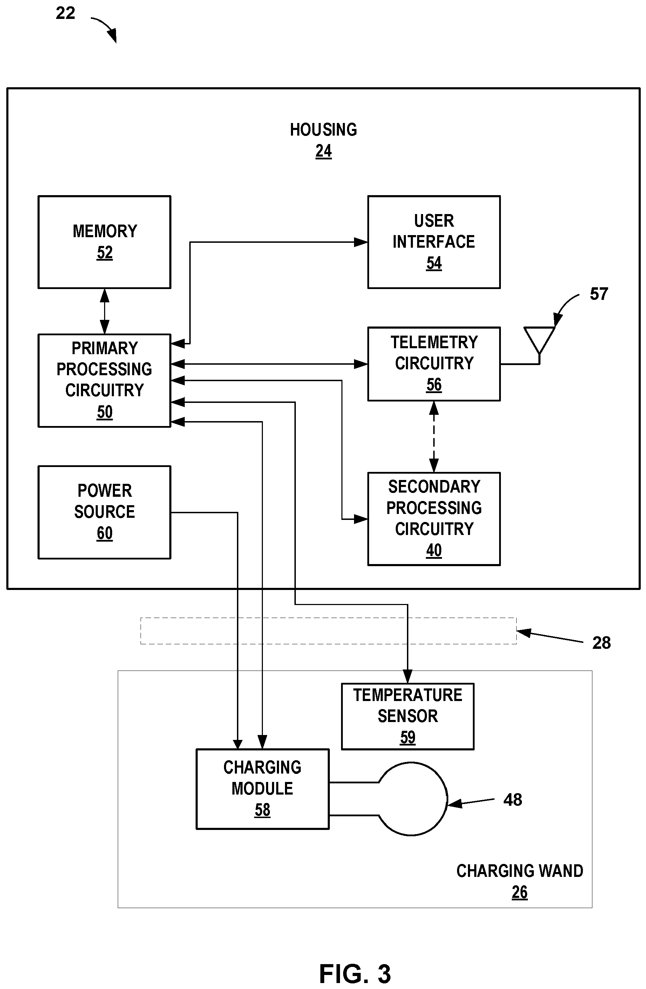

FIG. 3 is a block diagram of an example an external computing device of FIG. 1. External charging device 22 in of FIG. 2 is an example of external computing device 110 described above in relation to FIG. 1. In some examples, external charging device 22 may be described as a hand-held device, in other examples, external charging device 22 may be a larger or a non-portable device. In addition, in other examples external charging device 22 may be included as part of an external programmer or include functionality of an external programmer. As shown in the example of FIG. 3, external charging device 22 includes two separate components. Housing 24 encloses components such as a processing circuitry 50, memory 52, user interface 54, telemetry circuitry 56, and power source 60. Charging head 26, also called charging wand 26, may include charging module 58, temperature sensor 59, and coil 48. Housing 24 is electrically coupled to charging head 26 via charging cable 28.

A separate charging head 26 may facilitate optimal positioning of coil 48 over coil 16 of IMD 14. However, charging module 58 and/or coil 48 may be integrated within housing 24 in other examples. Memory 52 may store instructions that, when executed by processing circuitry 50, causes processing circuitry 50 and external charging device 22 to provide the functionality ascribed to external charging device 22 throughout this disclosure, and/or any equivalents thereof. Coil 48 may also be referred to as an antenna.

External charging device 22 may also include one or more temperature sensors, illustrated as temperature sensor 59, similar to temperature sensor 39 of FIG. 2. As shown in FIG. 3, temperature sensor 59 may be disposed within charging head 26. In other examples, one or more temperature sensors of temperature sensor 59 may be disposed within housing 24. For example, charging head 26 may include one or more temperature sensors positioned and configured to sense the temperature of coil 48 and/or a surface of the housing of charging head 26. In some examples, external charging device 22 may not include temperature sensor 59.

In general, external charging device 22 comprises any suitable arrangement of hardware, alone or in combination with software and/or firmware, to perform the techniques ascribed to external charging device 22, and processing circuitry 50, user interface 54, telemetry circuitry 56, and charging module 58 of external charging device 22, and/or any equivalents thereof. In various examples, external charging device 22 may include one or more processors, such as one or more microprocessors, DSPs, ASICs, FPGAs, or any other equivalent integrated or discrete logic circuitry, as well as any combinations of such components. External charging device 22 also, in various examples, may include a memory 52, such as RAM, ROM, PROM, EPROM, EEPROM, flash memory, a hard disk, a CD-ROM, comprising executable instructions for causing the one or more processors to perform the actions attributed to them. Moreover, although processing circuitry 50, telemetry circuitry 56, charging module 58, and temperature sensor 59 are described as separate modules, in some examples, processing circuitry 50, telemetry circuitry 56, charging module 58, and/or temperature sensor 59 are functionally integrated. In some examples, processing circuitry 50, telemetry circuitry 56, charging module 58, and/or temperature sensor 59 correspond to individual hardware units, such as ASICs, DSPs, FPGAs, or other hardware units.

Memory 52 may store instructions that, when executed by processing circuitry 50, cause processing circuitry 50 and external charging device 22 to provide the functionality ascribed to external charging device 22 throughout this disclosure, and/or any equivalents thereof. For example, memory 52 may include instructions that cause processing circuitry 50 to control the power level used to charge IMD 14 in response to the determined temperatures for the housing/external surface(s) of IMD 14, as communicated from IMD 14, or instructions for any other functionality. In addition, memory 52 may include a record of selected power levels, sensed temperatures, determined temperatures, or any other data related to charging rechargeable power source 18, described above in relation to FIG. 2.

Processing circuitry 50 may, when requested, transmit any of this stored data in memory 52 to another computing device for review or further processing, such as to servers 112 depicted in FIG. 1. Processing circuitry 50 may be configured to access memory, such as memory 32 of IMD 14 and/or memory 52 of external charging device 22, to retrieve information comprising instructions, formulas, and determined values for one or more constants.

User interface 54 may include a button or keypad, lights, a speaker for voice commands, a display, such as a liquid crystal (LCD), light-emitting diode (LED), or cathode ray tube (CRT). In some examples, the display may be a touch screen. As discussed in this disclosure, processing circuitry 50 may present and receive information relating to the charging of rechargeable power source 18 via user interface 54. For example, user interface 54 may indicate when charging is occurring, quality of the alignment between coils 40 and 48, the selected power level, current charge level of rechargeable power source 18, duration of the current recharge session, anticipated remaining time of the charging session, sensed temperatures, or any other information. Processing circuitry 50 may receive some of the information displayed on user interface 54 from IMD 14 in some examples. In some examples, user interface 54 may provide an indication to the user regarding the quality of alignment between coil 16, depicted in FIG. 2 and coil 48, based on the charge current to the battery.

User interface 54 may also receive user input via user interface 54. The input may be, for example, in the form of pressing a button on a keypad or selecting an icon from a touch screen. The input may change programmed settings, start or stop therapy, request starting or stopping a recharge session, a desired level of charging, or one or more statistics related to charging rechargeable power source 18 (e.g., the cumulative thermal dose). In this manner, user interface 54 may allow the user to view information related to the operation of IMD 14.

Charging module 58 may include one or more circuits that generate an electrical signal, and an electrical current, within primary coil 48. Charging module 58 may generate an alternating current of specified amplitude and frequency in some examples. In other examples, charging module 58 may generate a direct current. In any case, charging module 58 may be capable of generating electrical signals, and subsequent magnetic fields, to transmit various levels of power to IMD 14. In this manner, charging module 58 may be configured to charge rechargeable power source 18 of IMD 14 with the selected power level.

Power source 60 may deliver operating power to the components of external charging device 22. Power source 60 may also deliver the operating power to drive primary coil 48 during the charging process. Power source 60 may include a battery and a power generation circuit to produce the operating power. In some examples, a battery of power source 60 may be rechargeable to allow extended portable operation. In other examples, power source 60 may draw power from a wired voltage source such as a consumer or commercial power outlet.

Telemetry circuitry 56 supports wireless communication between IMD 14 and external charging device 22 under the control of processing circuitry 50. Telemetry circuitry 56 may also be configured to communicate with another computing device via wireless communication techniques, or direct communication through a wired connection. In some examples, telemetry circuitry 56 may be substantially similar to telemetry circuitry 36 of IMD 14 described herein, providing wireless communication via an RF or proximal inductive medium. In some examples, telemetry circuitry 56 may include an antenna 57, which may take on a variety of forms, such as an internal or external antenna. Although telemetry modules 56 and 36 may each include dedicated antennas for communications between these devices, telemetry modules 56 and 36 may instead, or additionally, be configured to utilize inductive coupling from coils 40 and 48 to transfer data.

Examples of local wireless communication techniques that may be employed to facilitate communication between external charging device 22 and IMD 14 include radio frequency and/or inductive communication according to any of a variety of standard or proprietary telemetry protocols, or according to other telemetry protocols such as the IEEE 802.11x or Bluetooth specification sets. In this manner, other external devices may be capable of communicating with external charging device 22 without needing to establish a secure wireless connection.

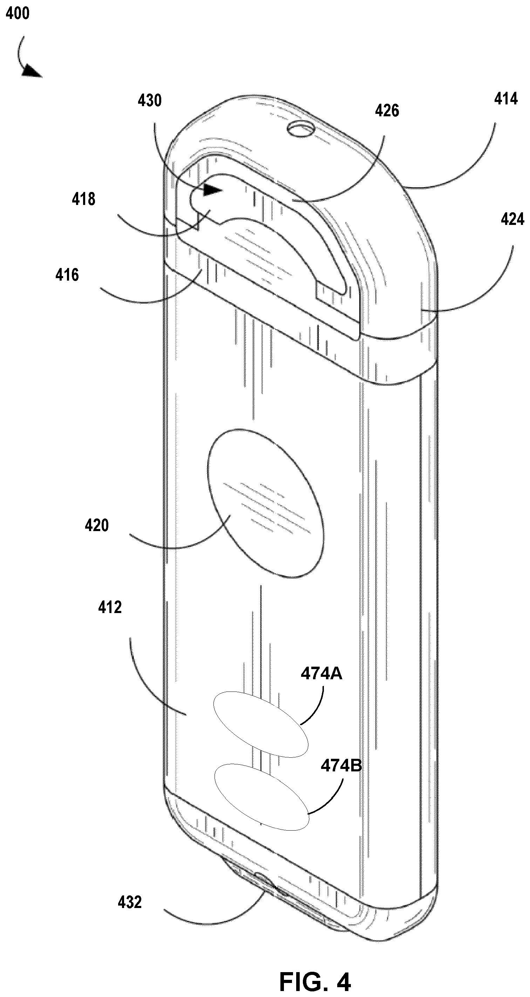

FIG. 4 is a conceptual diagram illustrating features of a medical device according to one or more techniques of this disclosure. Implantable medical device 400 in the example of FIG. 4 is a leadless neurostimulation device housing 412 enclosing components configured for delivering neurostimulation therapy. Implantable medical device 400 is an example of IMD 10 and IMD 14 described above in relation to FIGS. 1 and 2 respectively.

IMD 400 includes header unit 414 with one or more primary electrodes 418, and mounting plate 416 that couples housing 412 to header unit 414. In the example of FIG. 4, header unit 414 includes at least one primary electrode 418 that forms part of an exterior surface of header unit 414. Housing 412 includes a secondary electrode 420 that forms part of an exterior surface of housing 412 and is positioned on the same side of device 400 as primary electrode 418. In other examples, not depicted in FIG. 4, primary electrode 418 and secondary electrode 420 may be arranged on opposite sides of device 400. One or more suture elements 432 may be present on housing 412 to help fix device 400 in place after implantation.

Primary electrode 418 and secondary electrode 420 operate in conjunction with one another to provide stimulation therapy to a target treatment site (e.g., a tibial nerve or near a portion of the tibial nerve). Secondary electrode 420 may also be referred to as a case electrode, can electrode, or reference electrode. In some examples, primary electrode 418 may function as a cathode and secondary electrode 420 may function as an anode. In some examples, primary and secondary electrodes 418 and 420 may be characterized as a bipolar system. In this disclosure, the terms "primary" and "secondary" are used to differentiate two or more electrodes that are configured to transmit an electrical signal between the electrodes. The terms primary and/or secondary are not used to imply a hierarchy among the electrodes, positive and negative terminal, a total number of electrodes, or a directionality by which a signal is transmitted between the electrodes.

Header unit 414 includes outer housing 424, primary electrode 418, and dielectric mount 426. Outer housing 424 is coupled to mounting plate 416 and may define a partially recessed cavity that receives dielectric mount 426 and primary electrode 418. Outer housing 424 and mounting plate 416 may be made of metal or metal alloy (e.g., titanium or titanium alloy) to allow for easy coupling there between (e.g., laser welding) as well as allow for the coupling of mounting plate 416 to housing 412. Additionally, or alternatively, outer housing 424 or mounting plate 416 may be composed of a ceramic material, or nonconductive plastic material (e.g., polypropylene) including appropriate mechanisms (e.g., metal inserts) for coupling outer housing 424 to mounting plate 416.

In some examples, the seam between mounting plate 416 and outer housing 424 may form at least a partial hermetic seal. In an alternate example not depicted, header unit 414 may be configured so as to be mechanically coupled directly to housing 412, without the need for a separate mounting plate element.

Primary electrode 418 defines exterior contact surface 430 configured to be brought into direct contact with tissue of the patient. Contact surface 430 may also form a portion of a side of header unit 414, which may be on the same side of device 400 as secondary electrode 420. The material of the electrodes may depend on the type of signals to be measured and the electrical stimulation therapy to be delivered. In some examples, the electrodes of device 400 may be made from platinum, iridium and other combinations of materials. In some examples, forming rounded edges on electrodes may help avoid edge defects. In some examples, the position of the electrodes may be located on a single face of the device. An electrode of device 400 may be used as either a therapy electrode or a sensing electrode.

The exterior perimeter of contact surface 430 is at least partially bordered by dielectric mount 426, which may also form a portion of the exterior surface of header unit 414 absent of the dielectric coating disclosed below. Dielectric mount 426 electrically insulates and physically separates primary electrode 418 from outer housing 424, mounting plate 416, and other portions of device 400. Additionally, or alternatively, dielectric mount 426 may be molded around primary electrode 418 using silicone or liquid silicone rubber (LSR) for example, to help physically retain primary electrode 418 within header unit 414 of device 400. In some examples, dielectric mount 426 may be formed integrally with outer housing 424 provided the components are formed of a non-conductive material.

Outer housing 424 may form part the of the body of header unit 414. In particular, outer housing 424 forms the side of header unit 414 opposite of contact surface 430. Outer housing 424 may also form the perimeter edges (apart from the contact surface provided with mounting plate 416), and a portion of the same side of header unit 414 as contact surface 430 of primary electrode 418. In some examples, outer housing 424 may have a rounded, semicircular, or D-shaped perimeter edge that provides a relatively smooth surface without abrupt or sharp edges or lines than may present an irritation to the patient after implantation. In some examples, outer housing 424 is configured to receive and form a partial shell around dielectric mount 426. Outer housing 424 may define a concave interior surface (not shown in FIG. 4) that receives a portion of dielectric mount 426. Dielectric mount 426 may be secured to outer housing 424 using a suitable adhesive material (e.g., nonconductive medical adhesive, epoxy volcanized silicone, or the like).

Primary electrode 418 may be of suitable shape to provide electrical stimulation to the tibial nerve, such as through a fascia layer of a patient. In some examples, contact surface 430 of primary electrode 418 may be substantially flat (e.g., flat or nearly flat) as shown in FIG. 1. Alternatively, primary electrode 418 may define a curved surface (e.g., a semicylindrical shape or other 2D or 3D curved plane) that helps primary electrode 418 follow the curvatures of the fascia layer of a patient when implanted to provide better contact and focusing of the electrical signal directed to the tibial nerve. The curved surface may extend over the entirety of contact surface 430, or only over a portion of the surface. Additionally, or alternatively, the curvature may be confined to only contact surface 430 of primary electrode 418, or may extend over other portions of device 400 such as other parts of header 414, mounting plate 416, or housing 412. By including the curvature over other portions of device 400, the device may provide a more ergonomic fit when implanted while also helping to direct the stimulation signal to the tibial nerve.

In some examples, contact surface 430 of primary electrode 418 may also protrude from the plane defined by housing 412. Such a protrusion may help apply additional pressure to the fascia of the patient and help guide the electrical stimulation signal deeper into the tissue of the patient. Primary electrode 418 may also define one or more interlocking features, carveouts, recesses, or other structures that reduce the overall volume of primary electrode 418 without interfering with contact surface area 430. The reduced volume and interlocking features may also help reduce manufacturing costs as well as help fix primary electrode 418 relative to dielectric mount 426.

Primary electrode 418 may be formed using any suitable material capable of delivering electrical stimulation therapy to the patient once implanted. Such materials may include, but are not limited to titanium, titanium alloy, platinum iridium, or the like. In some examples, at least contact surface 430 is formed of platinum iridium, which provides low impedance to bodily tissue. The body of primary electrode 418 may be made of the same or different material than contact surface 430. For example, primary electrode 418 may be formed of titanium with contact surface 430 formed of platinum iridium. Using platinum iridium or titanium may be beneficial in reducing or eliminating the potential for charge buildup on the external surface of device 400 during operation.

Header unit 414 is coupled to mounting plate 416 and likewise mounting plate 416 is coupled to housing 412. Housing 412 includes secondary electrode 420. In some examples, secondary electrode 420 may be defined by an area of the body of housing 412. For example, housing 412 may be formed of a metallic material (e.g., titanium) and electrically coupled to the processing circuitry of leadless neurostimulation device 400. The outer surface of housing 412, including portions of mounting plate 416 and header unit 414, may be coated with a dielectric material apart from the surface area that defines secondary electrode 420 and primary electrode 418. The dielectric material may at least partially encapsulate device 400 such that the boundary created by the dielectric material define the area of secondary electrode 420, contact surface 430, or both.

Any of primary electrode 418, secondary electrode 420, and sensing electrodes 474A and 474B may connect to therapy circuitry 34 via electrodes 17A - 17D, described above in relation to FIG. 2. Sensing electrodes 474A and 474B may be configured to sense a bioelectrical signal, such as an EMG to detect the H-reflex elicited in response to an electrical stimulation therapy event, as described above in relation to FIGS. 1 - 3. Device 400 may use sensing electrodes 474A and 474B instead of, or along with an external sensor such as sensor 107 depicted in FIG. 1. The electrodes and other features in the example of FIG. 4 is just one example arrangement. In other examples, the location, size, spacing, shape, separation and other details of the electrodes may change. For example, one or more electrodes may be located on the opposite side of outer housing 424, or have a different shape or size than illustrated by the example of FIG. 4.

The dielectric coating may be applied using any suitable technique. In some such examples, the areas defining contact surface 430 and secondary electrode 420 may be masked with a suitable material such as tape. Leadless neurostimulation device 400 may be then coated using vapor deposition, dip coating, spray coating of similar technique with an adherent dielectric material followed by subsequent removal of the mask material to expose the surfaces of contact surface 430 and secondary electrode 420.

Suitable dielectric materials for coating leadless neurostimulation device 400 may include, but are not limited to, parylene, LSR, or silicone. Additionally, or alternatively, the outer surface of neurostimulation device 400 or portions thereof, may include a surface treatment such as an anodization treatment to modify portions of the surface to make the surface non-conductive. For example, portions of housing 412, outer housing 424, or both, if made of metal (e.g., titanium) may be treated through anodization to make select surfaces non-electrically conductive. In such examples, for purposes of this disclosure the exterior surface of the components may still be characterized as being metal (e.g., titanium) although the component has received such surface treatment.

In some examples, the outer surface of device 400 may be formed primarily of parylene. Formation of the desired electrode profiles may utilize dielectric blocking methods (e.g., use of a masking material during manufacture) or dielectric removal methods (e.g., removal via laser or soda blast) without damaging the dielectric coating.

In some examples, the dielectric coating may also contribute to creating a hermetic seal around leadless neurostimulation device 400. The configuration of attaching header 414 and housing 412 respectively to mounting plate 416 may also produce a hermetic seal within device 400. Coating device 400 with a dielectric material possessing sealing properties such as parylene, LSR, or silicone may provide additional robustness to the hermetic seal of device. Providing device 400 in a hermetically sealed form may contribute to the device's long-term functionality thereby providing advantages over other non-hermetically sealed devices.

The processing circuitry and components of neurostimulation device 400 are contained within housing 412 and not shown in FIG. 4. Examples of such processing components may include one or more electronic circuits for delivering electrical stimulation therapy, telemetry hardware, power supply, memory, processor(s). Housing 412 can also include communication circuitry disposed therein for receiving programming communication from an external programmer, or providing feedback to a programmer or other external device, as described above in relation to FIGS. 2 and 3.

In some examples, housing 412 may include an energy source e.g., a rechargeable or non-rechargeable battery. In other examples, device 400 can also be configured to receive energy signals from an external power transfer device, such as external computing device 110 described above in relation to FIG. 1. Device 400 may transduce the received energy signals into electrical power that is used to recharge a battery of the device. Additionally, or alternatively device 400 may include a non-rechargeable primary cell battery.

The size, shape, and physical separation distance between primary electrode 418 and secondary electrode 420, as well as of sensing electrodes 474A and 474B, may affect the functionality and effectiveness of device 400. In some examples, primary electrode 418 may define a contact surface area of about 5 mm² to about 90 mm². In some examples, device 400 may include only a single primary electrode 418 and the contact surface area of electrode 418 may be greater than about 10 mm², greater than about 15 mm², greater than about 18 mm², greater than about 20 mm², less than 35 mm², less than 30 mm², and less than 25 mm². In some examples, secondary electrode 420 may define a contact surface area of about 40 mm² to about 120 mm². However, examples of implantable medical devices having larger sized secondary electrodes may increase the current needed to create a therapeutic response. The separation distance between primary electrode 418 and secondary electrode 420 may be about 5 mm to about 15 mm. These dimensions are just some possible examples of electrode dimensions. Other electrode dimensions may apply based on type of therapy, placement of the device, target nerve, biological signals to be sensed and so on.

In some examples, the size, shape, and physical separation distance between primary electrode 418 and secondary electrode 420 may be configured to produce an impedance of less than 2,000 ohms (e.g., less than about 200 ohms). Additionally, or alternatively, primary and secondary electrodes 418 and 420 may be arranged in a non-concentric arrangement such that one electrode does not substantially encircle the other.

In other examples, header unit 414 may include a plurality of primary electrodes 418 (not shown in FIG. 4). In some examples, each primary electrode of the plurality of primary electrodes may be similarly sized and shaped. In other examples, the plurality of primary electrodes may include a collection of differently shaped and sized electrodes. In some examples, the one or more primary electrodes in header unit 414 may increase functionality and precision of device 400. For example, one or more of the primary electrodes may be configured to operate in one or more modes including one or more sensing modes where, for example, the electrode is used to detect measurable feedback from the tibial nerve (e.g., sensed activity or the nerve prior to or after stimulation) or sense the relative location of the tibial nerve to optimize stimulation and a delivery mode where the electrode delivers stimulation therapy to the tibial nerve. The processing circuitry may select one or more primary electrodes based on proximity to the tibial nerve for the delivery of stimulation therapy to steer the stimulation field.

Additionally, or alternatively, in a sensing mode, one or more of primary electrodes may be configured to monitor the activity of the tibial nerve or adjacent tissue prior to or during the delivery of simulation therapy, as described above in relation to FIGS. 1 - 3. For example, one or more primary electrodes may connect to electrodes 17A - 17D depicted in FIG. 2 and be configured to detect the stimulation artifact, an ECAP or some other bioelectrical signal. In some examples, one or more of the primary electrodes may be configured to detect the H-reflex to determine if sufficient therapy has been delivered, e.g., tibial nerve capture. The sensory mode may be actuated by processing circuitry contained in the body of housing 412.

In some examples, having multiple primary electrodes may improve stimulation targeting which could limit possible side effects from stimulating unintended areas. Improved targeting may also allow for reduced stimulation amplitudes which could improve battery longevity. Multiple primary electrodes may also provide unique therapy applications (e.g., providing stimulation to two sides of the nerve simultaneously) using one or multiple wave forms. Additionally, or alternatively the sensing technologies may be used for closed loop feedback, as described above in relation to FIGS. 1 - 3, e.g., to optimize stimulation (e.g., determine when to apply stimulation and when it is not needed, adjust parameters of the stimulation such as amplitude, voltage, or the like).

During operation, an electrical stimulation signal may be transmitted between primary electrode 418 and secondary electrode 420. The electrical signal may be used to stimulate tibial nerve which may be useful in the treatment of overactive bladder (OAB) symptoms of urinary urgency, urinary frequency and/or urge incontinence, or fecal incontinence, as described above in relation to FIG. 1.

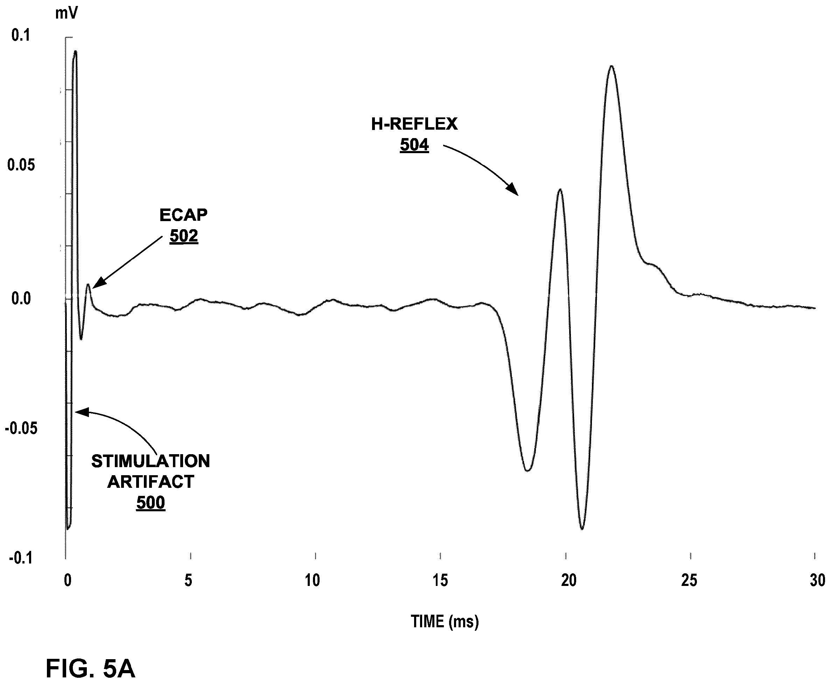

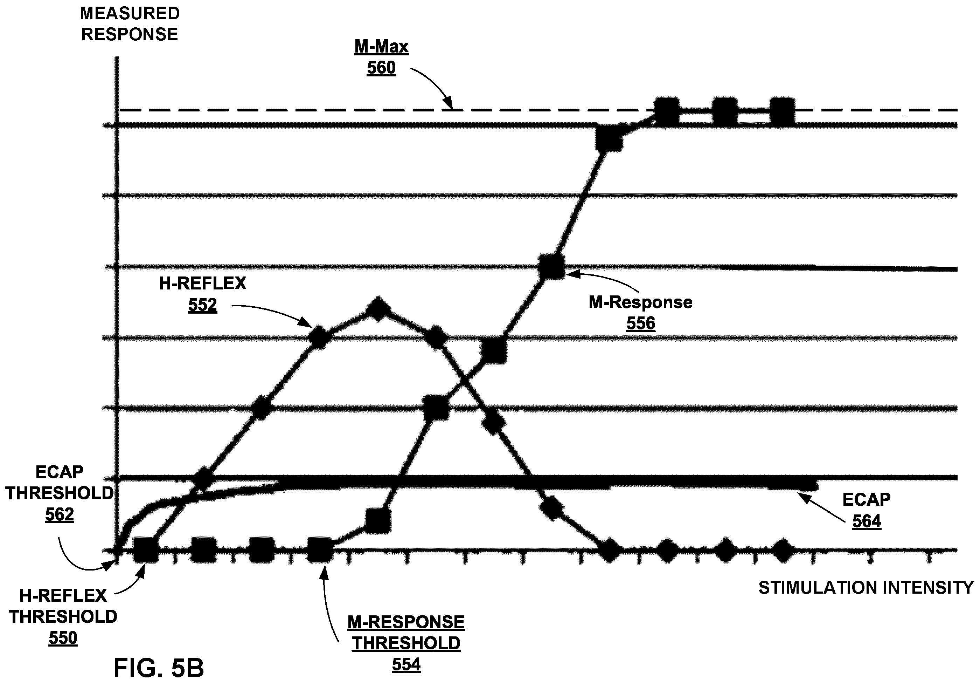

FIG. 5 is a timing diagram illustrating an example stimulation delivery and measured response according to one or more techniques of this disclosure. As described above in relation to FIGS. 1 - 4, an implantable medical device of this disclosure may deliver electrical stimulation therapy intended to capture a nerve, such as a tibial nerve depicted in FIG. 1. The device may deliver an electrical stimulation therapy event through one or more electrodes, e.g., electrodes 17A - 17D depicted in FIG. 2 and primary electrode 418 and secondary electrode 420 depicted in FIG. 4.

The delivered electrical stimulation therapy event may generate stimulation artifact 500. In some examples, the device may deliver an electrical stimulation therapy event with enough energy, e.g., a control pulse, to elicit ECAP 502. In some examples, electrodes connected to the implantable medical device may be configured to sense any or all of the stimulation artifact, an EMG and ECAPs 502. In some examples, the form factor/electrode size of the implantable medical device, such as device 400 described above in relation to FIG. 4, may make sensing and recording ECAPs difficult because the electrodes are large, and may not be in direct contact with the nerve. However, such an arrangement of electrodes, and/or the addition of sensing electrodes 474A and 474B may allow for capture of an EMG. In particular, sensing circuitry of the device may detect the EMG response related to H-reflex 504, that is directly related to the sensory fibers captured. As described above in relation to FIG. 1, processing circuitry of the device may determine whether the received indication of the motor response is valid based on the measured time interval after the delivery of the electrical stimulation therapy event. As shown in FIG. 5, H-reflex 504 is approximately twenty milliseconds after stimulation artifact 500. The specific timing may vary based on patient, location of the stimulation electrodes and location of the sensing device. For example, the timing window for the evoked H-reflex at sensing electrodes on the housing of the device may be different than for an external sensor, e.g., sensor 107 depicted in FIG. 1.

**In** some examples, measuring the magnitude of H-reflex 504 provides for closed loop control of stimulation. The processing circuitry of the device may compare characteristics of H-reflex 504, e.g., magnitude, shape, duration and so on, to one or more limits. Responsive to the magnitude, or other characteristic of the received indication of the motor response failing to satisfy the limit, the processing circuitry may cause the electrical stimulation circuitry to adjust at least one parameter (magnitude, pulse width and so on) of a subsequent electrical stimulation therapy event. In addition, over time monitoring H-reflex 504 may be a way to measure plasticity associated with effective therapy. As described above in relation to FIG. 1, an H-reflex, is as an evoked motor response that may be elicited by a first afferent stimulation and, through a synapse, an efferent alpha-motor neuron excitation.

**In** other examples, sensing circuitry of a medical device may measure characteristics of the M-wave to provide closed loop control of stimulation, similar to that described above for H-reflex 504. In other examples, the medical device may output electrical stimulation at a level that causes only an ECAP response, e.g., indicating sensory nerve capture but without the motor component. In some examples, the IMD of this disclosure may be configured to capture either or both an M-wave and H-reflex 504.

As described above in relation to FIG. 1, the medical device of this disclosure may operate in different closed loop modes. In some examples, the medical device may operate in a single mode for a particular patient. In other examples, the medical device may operate in one mode for a first duration and a second mode for another duration for the same patient. As noted above, one other example mode may be a mode in which, the medical device may output electrical stimulation just below stimulation level that would generate a motor response. Said another way, the device may output electrical stimulation as high as possible without generating a significant motor component. In general, responsive to the magnitude, or other characteristic of the received indication of the H-reflex, an M-wave, or an ECAP satisfying, or failing to satisfy a respective limit, the processing circuitry may cause the electrical stimulation circuitry to adjust at least one parameter (magnitude, pulse width and so on) of a subsequent electrical stimulation therapy event. In other words, processing circuitry of the medical device may measure and analyze some combination of the H-reflex, an M-wave, or ECAP to provide closed loop control of stimulation.

FIG. 5B is a graph illustrating how the measured response may change based on the stimulation intensity. The shape of the curve, and relative values, may change from patient to patient, and may change over time for a particular patient.

An IMD, such as IMD 10 depicted in FIG. 1, may deliver a sufficient short-duration, low-intensity electric stimulus to the tibial nerve, the electrical stimulus may elicit action potentials in type Ia sensory afferents because sensory afferent axons such as sensory afferent axon 102 may have a large axon diameter. As described above in relation to FIG. 1, for the H-reflex pathway 552, these action potentials may travel to the spinal cord, where the action potential may cause excitatory postsynaptic potentials, in turn eliciting action potentials, which travel down the alpha motor neuron (αMN) axons toward a muscle. As described above, alpha-motor neurons are the nerve fibers that directly innervate skeletal muscles to initiate contractions. A sensor, e.g., sensor 107 described above in relation to FIG. 1, or other sensing circuitry connected to electrodes, described above in relation to FIG. 4, may detect and measure a reflex response in the muscle as an H-reflex.

Increasing the stimulus intensity of the electrical stimulation therapy event may cause action potentials to occur in the thinner axons of the αMNs. The H-reflex threshold 550 is less than the M-response threshold 554 in the example of FIG. 5B. The ECAP threshold 562 is less than both the H-reflex threshold 550 and the M-response threshold 554 in the example of FIG. 5B. As noted above, stimulation intensity may refer to any combination of changes in stimulation current magnitude, pulse width, number of pulses in a burst, and so on. The stimulation intensity for a therapy event may be tuned so that that the therapy event exceeds the nerve capture threshold and stimulates sensory fibers of the nerve.

For the M-wave pathway, the action potentials may travel directly toward the muscle and be recorded as the M-response 556, which may also be referred to as an M-wave. In some examples, and the same time, action potentials from the higher intensity stimulus may propagate antidromically (backward) in the αMN toward the spinal cord and may collide with action potentials of the evoked H-reflex response 552, which may result in partial cancellation of the reflex response. As the stimulation intensity increases, orthodromic (toward the muscle) and antidromic (toward the spinal cord) action potentials may occur in more MN axons, e.g., for stimulation intensities more than M-response threshold 554. As stimulation intensity increases further, orthodromic (toward the muscle) potentials may reach a maximum (Mmax 560), while antidromic (toward the spinal cord) action potentials 552 may results in complete cancellation of the H-reflex.

As described above in relation to FIG. 1, afferent stimulation, in the context of nerves means conducting toward the central nervous system. The term efferent means 'away' and may typically be used for motor fibers. In some examples, afferent may be used synonymously with sensory since the sensory fibers convey sensations back to the spinal cord/brain.

In some examples, the closed loop functions of system 100 may be desirable to deliver electrical stimulation therapy at a sufficient intensity to provide a therapeutic effect that eases the patient's symptoms, yet with a stimulation intensity (e.g., magnitude, pulse width, pulse repetition rate, and so on) to elicit a measurable H-reflex (e.g., the M-wave does not cancel the H-reflex). In addition, delivering electrical stimulation therapy at lower levels may reduce power consumption and therefore a battery of the IMD may last longer, or need less recharging, which may be beneficial for the patient.

In other examples, as described above in relation to FIG. 1, the processing circuitry of an IMD of this disclosure may receive an indication of an H-reflex above a predetermined limit stored at a memory location, e.g., a limit of greater than zero. The processing circuitry may adjust parameters of the electrical stimulation therapy event to ensure that the therapy event does not elicit an H-reflex, but does elicit an afferent response. Said another way, the processing circuitry may adjust the parameters of a subsequent therapy event to be above the capture threshold for an afferent response (e.g., elicit an afferent response) but less than the capture threshold for an H-reflex that comes from the spinal cord.

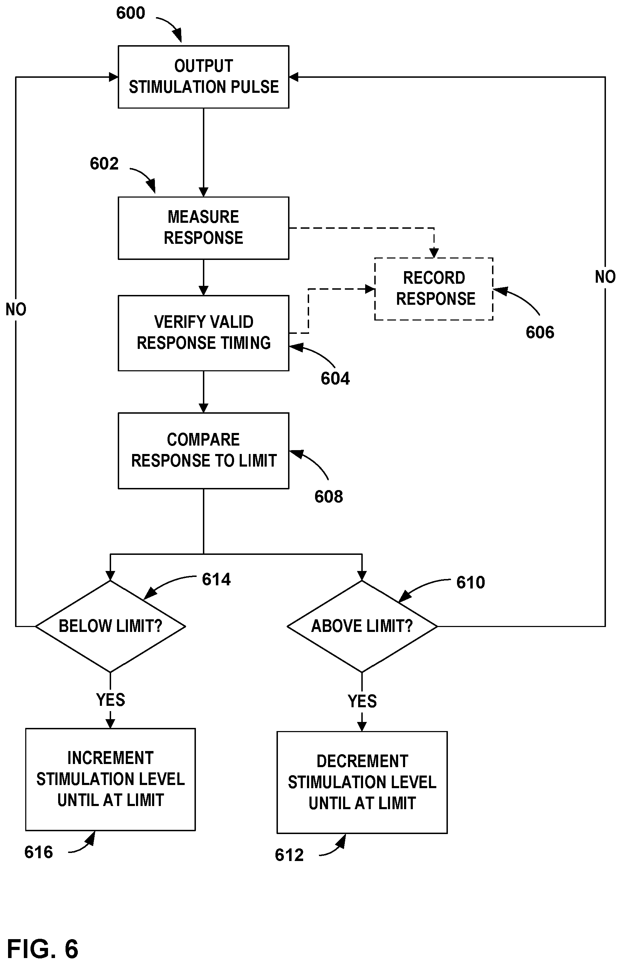

FIG. 6 is a flow chart illustrating an example operation of the system of this disclosure. The blocks of FIG. 6 will be described in terms of FIGS. 1 and 2, unless otherwise noted.

IMD 10, shown in FIG. 1, may deliver electrical stimulation therapy, e.g., a stimulation pulse (600) to a target nerve of a patient via electrical stimulation circuitry such as therapy circuitry 34 and electrodes 17A - 17D. In some examples, one or more of electrodes may be located on the housing of IMD 10, as shown in FIG. 4 for electrodes 418 and 420. Processing circuitry 30 may cause therapy circuitry 34 to deliver a stimulation pulse with sufficient intensity such that electrical stimulation therapy event causes an afferent stimulation in the target nerve. As described above in relation to FIGS. 1 and 5B, an electrical stimulation therapy event with an intensity that is too low (below a stimulation threshold) may not capture the target nerve. An electrical stimulation therapy event with an intensity that is too high (above a stimulation threshold) may cancel the evoked motor response.

Processing circuitry 30 may receive an indication from at least one sensor indicating a motor response of the patient (602). Sensing circuitry, e.g., therapy circuitry 34, may connect to electrodes on the housing of IMD 14. In other examples, processing circuitry 30 may receive an indication from a remote sensor, e.g., sensor 107, via wired or wireless connection, such as via telemetry circuitry 36. The motor response measured by the at least one sensor is an evoked motor response caused by the electrical stimulation therapy event.

Because the evoked motor response is an efferent alpha motor neuron excitation through a spinal synapse, processing circuitry 30 may verify the timing of evoked response by measuring a time interval between the delivered electrical stimulation therapy from the electrical stimulation circuitry and the received indication of the motor response (604). In some examples, processing circuitry 30 may record the measured response (606) after measuring the response (602), e.g., at memory 32. In other examples, processing circuitry 30 may first determine whether the received indication of the motor response is valid based on the measured time interval (604) before storing the measured response (606).

Responsive to determining that the motor response is valid, processing circuitry 30 may determine whether a magnitude of the received indication of the motor response satisfies a limit (608). In the example of FIG. 6, the predetermined limit may be considered a programming limit value, or range, e.g., a range between an upper limit and low limit. In other words, processing circuitry 30 may compare a measured value to a limit value stored in memory 32. The limit values stored in memory 32 may include the upper and lower limits of a range. Processing circuitry 30 may compare any one or more characteristics to one or more limits to determine whether the electrical stimulation therapy event sufficiently captured the target nerve to provide a therapeutic effect (e.g., for incontinence, or other symptoms). As depicted in FIG. 5, in some examples, the processing circuitry may compare a voltage magnitude and/or a voltage difference between a maximum and minimum voltages of H-reflex 504 to a voltage magnitude limit. In other examples, processing circuitry 30 may calculate an intensity, or amount of energy carried by H-reflex 504, e.g., by an integration of the voltage magnitude over time. In other examples, processing circuitry 30 may measure the duration, pulse width, shape, number of peaks, ratio of peak amplitude(s), signal to noise ratio, width of peak(s), latency of peaks(s) or other characteristics of H-reflex 504. Some other characteristics may also include: H-reflex magnitude, latency, intensity, H-reflex latency compared to stim or compared to an ECAP, the capture threshold of H-reflex compared to the capture threshold of ECAP, ECAP magnitude, latency, intensity at H-reflex, M-wave magnitude latency, M-wave intensity, ratio of M-wave/H-reflex capture threshold, magnitude, latency, intensity, ratio of H-reflex/ECAP threshold magnitude latency intensity, ratio of M-wave/ECAP threshold magnitude latency intensity, and so on.

Responsive to the magnitude of the received indication of the motor response failing to satisfy the limit (610 and 614), processing circuitry 30 may cause the electrical stimulation circuitry, e.g., therapy circuitry 34, to adjust at least one parameter of a subsequent electrical stimulation therapy event. Examples of adjustments may include voltage magnitude, current magnitude, pulse width, number of pulses and so on, as described above in relation to FIG. 1. When the measured response is within the limits, (NO branches of 610 and 614), processing circuitry 30 may cause no adjustments and output a subsequent electrical stimulation therapy event with the same characteristics.

As one possible example, when the measured evoked α-MN response is less than a predetermined limit (YES branch of 614), processing circuitry 30 may cause therapy circuitry 34 to increment a stimulation level, until the evoked α-MN response is above the predetermined limit (616). When the measured evoked α-MN response is above a second predetermine limit (YES branch of 610), processing circuitry 30 may cause therapy circuitry 34 to decrement a stimulation level (616), until the evoked α-MN response is above the predetermined limit. In some examples, therapy circuitry 34 may decrement the stimulation level by reducing a voltage magnitude, reducing a pulse width, and so on.

In other examples, the values for the limit stored at the memory may be set to one or more nerve capture thresholds. For example, as depicted in FIG. 5B, processing circuitry 30 may increment the stimulation level (616) until the stimulation level exceeds the H-reflex threshold 550. In other words, above stimulation intensity that therapy and sensing circuitry 34 begins to measure an H-reflex. Processing circuitry may decrement the stimulation level 612, to ensure the stimulation intensity is less than the M-response threshold 554. In other words, such that the stimulation therapy does not evoke a measurable M-response 556, e.g., M-response 556 level is less than a limit stored at memory 32.

In one or more examples, the functions described above may be implemented in hardware, software, firmware, or any combination thereof. For example, the various components of FIGS. 1 - 3, e.g., processing circuitry of IMD 10 or processing circuitry of external computing device 110, or some combination of IMD 10, external computing device 110 and servers 112, may perform any of the functions described above. Such components may be implemented in hardware, software, firmware, or any combination thereof. If implemented in software, the functions may be stored on or transmitted over, as one or more instructions or code, a computer-readable medium and executed by a hardware-based processing unit. Computer-readable media may include computer-readable storage media, which corresponds to a tangible medium such as data storage media, or communication media including any medium that facilitates transfer of a computer program from one place to another, e.g., according to a communication protocol. In this manner, computer-readable media generally may correspond to (1) tangible computer-readable storage media which is non-transitory or (2) a communication medium such as a signal or carrier wave. Data storage media may be any available media that can be accessed by one or more computers or one or more processors to retrieve instructions, code and/or data structures for implementation of the techniques described in this disclosure. A computer program product may include a computer-readable medium.

The term "non-transitory" may indicate that the storage medium is not embodied in a carrier wave or a propagated signal. In certain examples, a non-transitory storage medium may store data that can, over time, change (e.g., in RAM or cache). By way of example, and not limitation, such computer-readable storage media, may include random access memory (RAM), read only memory (ROM), programmable read only memory (PROM), erasable programmable read only memory (EPROM), electronically erasable programmable read only memory (EEPROM), flash memory, a hard disk, a compact disc ROM (CD-ROM), a floppy disk, a cassette, magnetic media, optical media, or other computer readable media. In some examples, an article of manufacture may include one or more computer-readable storage media.

Also, any connection is properly termed a computer-readable medium. For example, if instructions are transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of medium. It should be understood, however, that computer-readable storage media and data storage media do not include connections, carrier waves, signals, or other transient media, but are instead directed to non-transient, tangible storage media. Combinations of the above should also be included within the scope of computer-readable media.

Instructions may be executed by one or more processors, such as one or more DSPs, general purpose microprocessors, ASICs, FPGAs, or other equivalent integrated or discrete logic circuitry. Accordingly, the term "processor," as used herein, such as processing circuitry 30 of IMD 10, processing circuitry 50 of external computing device 110, and of servers 112, may refer to any of the foregoing structure or any other structure suitable for implementation of the techniques described herein. Also, the techniques could be fully implemented in one or more circuits or logic elements.

The techniques of this disclosure may be implemented in a wide variety of devices or apparatuses, including, an integrated circuit (IC) or a set of ICs (e.g., a chip set). Various components, modules, or units are described in this disclosure to emphasize functional aspects of devices configured to perform the disclosed techniques, but do not necessarily require realization by different hardware units. Rather, as described above, various units may be combined in a hardware unit or provided by a collection of interoperative hardware units, including one or more processors as described above, in conjunction with suitable software and/or firmware.

## Claims

1. A device comprising:
electrical stimulation circuitry (34) configured to output electrical stimulation therapy to a target nerve of a patient; and
processing circuitry (30) operatively coupled to the electrical stimulation circuitry (34), the processing circuitry configured to:
receive an indication from at least one sensor indicating a motor response;
measure a time interval between an output of an electrical stimulation therapy event from the electrical stimulation circuitry (34) and the received indication of the motor response;
determine whether the received indication of the motor response is valid based on the measured time interval;
responsive to determining that the motor response is valid, determine whether a magnitude of the received indication of the motor response satisfies a target; and
responsive to the magnitude of the received indication of the motor response failing to satisfy the target, cause the electrical stimulation circuitry (34) to adjust at least one parameter of a subsequent electrical stimulation therapy event.

2. The device of claim 1,
wherein the device comprises a housing (19);
wherein the device comprises at least one electrode attached to the housing (19) configured to be in contact with tissue of the patient; and
wherein the at least one electrode (17) delivers the electrical stimulation therapy to the target nerve of the patient.

3. The device of any of the preceding claims, wherein the at least one sensor is configured to be attached to a housing that is in contact with tissue of the patient and that includes the electrical stimulation circuitry (34).

4. The device of any of the preceding claims, wherein the at least one sensor is configured to measure an H-reflex as reflex muscle response recorded from a muscle.

5. The device of any of the preceding claims,
wherein the motor response measured by the at least one sensor is an evoked motor response caused by the electrical stimulation therapy event; and
wherein the evoked motor response is an efferent alpha motor neuron excitation through a spinal synapse.

6. The device of any of the preceding claims, wherein responsive to the magnitude of the received indication of the motor response being less than the target, cause the electrical stimulation circuitry to increase a stimulation level of the subsequent electrical stimulation therapy event.

7. A system comprising:
the device of claim 1; and
the at least one sensor (107) configured to measure a motor response of a patient.

8. The system of claim 7,
wherein the device comprises a housing (19);
wherein the device comprises at least one electrode (17) attached to the housing (19) configured to be in contact with tissue of the patient; and
wherein the at least one electrode (17) is configured to deliver the electrical stimulation therapy to the target nerve of the patient.

9. The system of any of the claims 7 or 8, wherein the at least one sensor (107) is external to and separate from a housing that includes the electrical stimulation circuitry (34).

10. The system of any of the claims 7 to 9, wherein the at least one sensor is a wearable sensor and/or wherein the at least one sensor is attached to a housing that is in contact with tissue of the patient and that includes the electrical stimulation circuitry (34).

11. The system of any of the claims 7 to 10, wherein the at least one sensor (107) is configured to measure an H-reflex as a reflex muscle response recorded from a muscle.

12. The system of any of the claims 7 to 11, wherein electrical stimulation therapy event causes an afferent response in the target nerve, in particular, wherein the motor response measured by the at least one sensor is an evoked motor response caused by the electrical stimulation therapy event.

13. The system of any of the claims 7 to 12, wherein responsive to the magnitude of the received indication of the motor response being less than the target, cause the electrical stimulation circuitry (34) to increase a stimulation level of the subsequent electrical stimulation therapy event.

14. The system of any of the claims 7 to 13, wherein the at least one sensor (107) is configured to measure at least one electromyography (EMG) response.

15. A computer-readable storage medium storing instruction thereon that when executed cause one or more processors to perform a method comprising:
delivering, by electrical stimulation circuitry of an implantable medical device, electrical stimulation therapy to a target nerve of a patient;
receiving, by processing circuitry of the implantable medical device, an indication from at least one sensor indicating a motor response of the patient;
measuring, by the processing circuitry, a time interval between the delivered electrical stimulation therapy from the electrical stimulation circuitry and the received indication of the motor response;
determining, by the processing circuitry, whether the received indication of the motor response is valid based on the measured time interval;
responsive to determining that the motor response is valid, determining, by the processing circuitry, whether a magnitude of the received indication of the motor response satisfies a target; and
responsive to the magnitude of the received indication of the motor response failing to satisfy the target, causing, by the processing circuitry, the electrical stimulation circuitry to adjust at least one parameter of a subsequent electrical stimulation therapy event.

## Patentansprüche

1. Vorrichtung, umfassend:
eine elektrische Stimulationsschaltung (34), die dazu konfiguriert ist, eine elektrische Stimulationstherapie an einen Zielnerv eines Patienten abzugeben; und
eine Verarbeitungsschaltung (30), die mit der elektrischen Stimulationsschaltung (34) wirkgekoppelt ist, wobei die Verarbeitungsschaltung konfiguriert ist zum:
Empfangen einer Angabe von mindestens einem Sensor, die eine motorische Reaktion angibt;
Messen eines Zeitintervalls zwischen einer Ausgabe eines elektrischen Stimulationstherapieereignisses von der elektrischen Stimulationsschaltung (34) und der empfangenen Angabe der motorischen Reaktion;
Bestimmen, ob die empfangene Angabe der motorischen Reaktion basierend auf dem gemessenen Zeitintervall gültig ist;
als Reaktion auf das Bestimmen, dass die motorische Reaktion gültig ist, Bestimmen, ob eine Größe der empfangenen Angabe der motorischen Reaktion einen Zielwert erfüllt; und
als Reaktion darauf, dass die Größe der empfangenen Angabe der motorischen Reaktion den Zielwert nicht erfüllt, Veranlassen der elektrischen Stimulationsschaltung (34), mindestens einen Parameter eines nachfolgenden elektrischen Stimulationstherapieereignisses anzupassen.

2. Vorrichtung nach Anspruch 1,
wobei die Vorrichtung ein Gehäuse (19) umfasst;
wobei die Vorrichtung mindestens eine an dem Gehäuse (19) befestigte Elektrode umfasst, die konfiguriert ist, um in Kontakt mit Gewebe des Patienten zu stehen; und
wobei die mindestens eine Elektrode (17) die elektrische Stimulationstherapie an den Zielnerv des Patienten abgibt.

3. Vorrichtung nach einem der vorstehenden Ansprüche, wobei der mindestens eine Sensor konfiguriert ist, um an einem Gehäuse befestigt zu werden, das in Kontakt mit Gewebe des Patienten steht und das die elektrische Stimulationsschaltung (34) einschließt.

4. Vorrichtung nach einem der vorstehenden Ansprüche, wobei der mindestens eine Sensor dazu konfiguriert ist, einen H-Reflex als Reflexmuskelreaktion zu messen, die von einem Muskel registriert wird.

5. Vorrichtung nach einem der vorstehenden Ansprüche,
wobei die durch den mindestens einen Sensor gemessene motorische Reaktion eine durch das elektrische Stimulationstherapieereignis hervorgerufene motorische Reaktion ist; und
wobei die hervorgerufene motorische Reaktion eine efferente Alpha-Motoneuronen-Erregung durch eine spinale Synapse ist.

6. Vorrichtung nach einem der vorstehenden Ansprüche, wobei als Reaktion darauf, dass die Größe der empfangenen Angabe der motorischen Reaktion unter dem Zielwert liegt, die elektrische Stimulationsschaltung veranlasst wird, einen Stimulationspegel des nachfolgenden elektrischen Stimulationstherapieereignisses zu erhöhen.

7. System, umfassend:
die Vorrichtung nach Anspruch 1; und
den mindestens eine Sensor (107), der dazu konfiguriert ist, eine motorische Reaktion eines Patienten zu messen.

8. System nach Anspruch 7,
wobei die Vorrichtung ein Gehäuse (19) umfasst;
wobei die Vorrichtung mindestens eine an dem Gehäuse (19) befestigte Elektrode (17) umfasst, die konfiguriert ist, um in Kontakt mit Gewebe des Patienten zu stehen; und
wobei die mindestens eine Elektrode (17) dazu konfiguriert ist, die elektrische Stimulationstherapie an den Zielnerv des Patienten abzugeben.

9. System nach einem der Ansprüche 7 oder 8, wobei der mindestens eine Sensor (107) außerhalb zu und getrennt von einem Gehäuse angeordnet ist, das die elektrische Stimulationsschaltung (34) einschließt.

10. System nach einem der Ansprüche 7 bis 9, wobei der mindestens eine Sensor ein tragbarer Sensor ist und/oder wobei der mindestens eine Sensor an einem Gehäuse befestigt ist, das in Kontakt mit Gewebe des Patienten steht und das die elektrische Stimulationsschaltung (34) einschließt.

11. System nach einem der Ansprüche 7 bis 10, wobei der mindestens eine Sensor (107) dazu konfiguriert ist, einen H-Reflex als Reflexmuskelreaktion zu messen, die von einem Muskel registriert wird.

12. System nach einem der Ansprüche 7 bis 11, wobei das elektrische Stimulationstherapieereignis eine afferente Reaktion in dem Zielnerv verursacht, insbesondere wobei die von dem mindestens einen Sensor gemessene motorische Reaktion eine durch das elektrische Stimulationstherapieereignis hervorgerufene motorische Reaktion ist.

13. System nach einem der Ansprüche 7 bis 12, wobei als Reaktion darauf, dass die Größe der empfangenen Angabe der motorischen Reaktion unter dem Zielwert liegt, die elektrische Stimulationsschaltung (34) veranlasst wird, einen Stimulationspegel des nachfolgenden elektrischen Stimulationstherapieereignisses zu erhöhen.

14. System nach einem der Ansprüche 7 bis 13, wobei der mindestens eine Sensor (107) dazu konfiguriert ist, mindestens eine Elektromyographie(EMG)-Reaktion zu messen.

15. Computerlesbares Speichermedium, das Anweisungen darauf speichert, die, wenn sie ausgeführt werden, einen oder mehrere Prozessoren veranlassen, ein Verfahren durchzuführen, das umfasst:
Abgeben einer elektrischen Stimulationstherapie an einen Zielnerv eines Patienten durch eine elektrische Stimulationsschaltung einer implantierbaren medizinischen Vorrichtung;
Empfangen, durch eine Verarbeitungsschaltung der implantierbaren medizinischen Vorrichtung, einer Angabe von mindestens einem Sensor, die eine motorische Reaktion des Patienten angibt;
Messen, durch die Verarbeitungsschaltung, eines Zeitintervalls zwischen der abgegebenen elektrischen Stimulationstherapie von dem elektrischen Stimulationsschaltkreis und der empfangenen Angabe der motorischen Reaktion;
Bestimmen, durch die Verarbeitungsschaltung, ob die empfangene Angabe der motorischen Reaktion basierend auf dem gemessenen Zeitintervall gültig ist;
als Reaktion auf das Bestimmen, dass die motorische Reaktion gültig ist, Bestimmen, durch die Verarbeitungsschaltung, ob eine Größe der empfangenen Angabe der motorischen Reaktion einen Zielwert erfüllt; und
als Reaktion darauf, dass die Größe der empfangenen Angabe der motorischen Reaktion den Zielwert nicht erfüllt, Veranlassen, durch die Verarbeitungsschaltung, der elektrischen Stimulationsschaltung, mindestens einen Parameter eines nachfolgenden elektrischen Stimulationstherapieereignisses anzupassen.

## Revendications

1. Dispositif comprenant :
un ensemble de circuits de stimulation électrique (34) configurés pour délivrer en sortie une thérapie de stimulation électrique à un nerf cible d'un patient ; et
un ensemble de circuits de traitement (30) couplés de manière fonctionnelle à l'ensemble de circuits de stimulation électrique (34), l'ensemble de circuits de traitement configurés pour :
recevoir une indication d'au moins un capteur indiquant une réponse motrice ;
mesurer un intervalle de temps entre une sortie d'un événement de thérapie de stimulation électrique provenant de l'ensemble de circuits de stimulation électrique (34) et l'indication reçue de la réponse motrice ;
déterminer si l'indication reçue de la réponse motrice est valide sur la base de l'intervalle de temps mesuré ;
en réponse à la détermination que la réponse motrice est valide, déterminer si une amplitude de l'indication reçue de la réponse motrice satisfait une cible ; et
en réponse à l'ampleur de l'indication reçue de la réponse motrice ne satisfaisant pas la cible, amener l'ensemble de circuits de stimulation électrique (34) à ajuster au moins un paramètre d'un événement de thérapie de stimulation électrique ultérieur.

2. Dispositif selon la revendication 1,
dans lequel le dispositif comprend un boîtier (19) ;
dans lequel le dispositif comprend au moins une électrode fixée au boîtier (19) conçue pour être en contact avec un tissu du patient ; et
dans lequel l'au moins une électrode (17) administre la thérapie de stimulation électrique au nerf cible du patient.

3. Dispositif selon l'une quelconque des revendications précédentes, dans lequel l'au moins un capteur est configuré pour être fixé à un boîtier qui est en contact avec un tissu du patient et qui comporte l'ensemble de circuits de stimulation électrique (34).

4. Dispositif selon l'une quelconque des revendications précédentes, dans lequel l'au moins un capteur est configuré pour mesurer un réflexe H en tant que réponse musculaire réflexe enregistrée à partir d'un muscle.

5. Dispositif selon l'une quelconque des revendications précédentes,
dans lequel la réponse motrice mesurée par l'au moins un capteur est une réponse motrice évoquée causée par l'événement de thérapie de stimulation électrique ; et
dans lequel la réponse motrice évoquée est une excitation efférente par motoneurone alpha à travers une synapse spinale.

6. Dispositif selon l'une quelconque des revendications précédentes, dans lequel, en réponse à l'amplitude de l'indication reçue de la réponse motrice inférieure à la cible, amener l'ensemble de circuits de stimulation électrique à augmenter un niveau de stimulation de l'événement de thérapie de stimulation électrique ultérieur.

7. Système comprenant :
le dispositif selon la revendication 1 ; et
l'au moins un capteur (107) configuré pour mesurer une réponse motrice d'un patient.

8. Système selon la revendication 7,
dans lequel le dispositif comprend un boîtier (19) ;
dans lequel le dispositif comprend au moins une électrode (17) fixée au boîtier (19) conçue pour être en contact avec le tissu du patient ; et
dans lequel l'au moins une électrode (17) est configurée pour administrer la thérapie de stimulation électrique au nerf cible du patient.

9. Système selon l'une quelconque des revendications 7 ou 8, dans lequel l'au moins un capteur (107) est externe et séparé d'un boîtier qui comporte l'ensemble de circuits de stimulation électrique (34).

10. Système selon l'une quelconque des revendications 7 à 9, dans lequel l'au moins un capteur est un capteur portable et/ou dans lequel l'au moins un capteur est fixé à un boîtier qui est en contact avec le tissu du patient et qui comporte l'ensemble de circuits de stimulation électrique (34).

11. Système selon l'une quelconque des revendications 7 à 10, dans lequel l'au moins un capteur (107) est configuré pour mesurer un réflexe H en tant que réponse musculaire réflexe enregistrée à partir d'un muscle.

12. Système selon l'une quelconque des revendications 7 à 11, dans lequel un événement de thérapie de stimulation électrique provoque une réponse afférente dans le nerf cible, en particulier, dans lequel la réponse motrice mesurée par l'au moins un capteur est une réponse motrice évoquée causée par l'événement de thérapie de stimulation électrique.

13. Système selon l'une quelconque des revendications 7 à 12, dans lequel, en réponse à l'amplitude de l'indication reçue de la réponse motrice étant inférieure à la cible, l'ensemble de circuits de stimulation électrique (34) à augmentent un niveau de stimulation de l'événement de thérapie de stimulation électrique ultérieur.

14. Système selon l'une quelconque des revendications 7 à 13, dans lequel l'au moins un capteur (107) est configuré pour mesurer au moins une réponse d'électromyographie (EMG).

15. Support de stockage lisible par ordinateur stockant des instructions sur celui-ci, qui lorsqu'elles sont exécutées amènent un ou plusieurs processeurs à effectuer un procédé comprenant :
l'administration, par un ensemble de circuits de stimulation électrique d'un dispositif médical implantable, d'une thérapie de stimulation électrique à un nerf cible d'un patient ;
la réception, par un ensemble de circuits de traitement du dispositif médical implantable, d'une indication provenant d'au moins un capteur indiquant une réponse motrice du patient ;
la mesure, par l'ensemble de circuits de traitement, d'un intervalle de temps entre la thérapie de stimulation électrique administrée par l'ensemble de circuits de stimulation électrique et l'indication reçue de la réponse motrice ;
la détermination, par l'ensemble de circuits de traitement, du fait de savoir si l'indication reçue de la réponse motrice est valide sur la base de l'intervalle de temps mesuré ;
en réponse à la détermination que la réponse motrice est valide, la détermination, par l'ensemble de circuits de traitement, du fait de déterminer si une amplitude de l'indication reçue de la réponse motrice satisfait une cible ; et
en réponse à l'ampleur de l'indication reçue de la réponse motrice ne satisfaisant pas la cible, le fait d'amener, par l'ensemble de circuits de traitement, l'ensemble de circuits de stimulation électrique à ajuster au moins un paramètre d'un événement de thérapie de stimulation électrique ultérieur.
